# EUROPEAN PATENT APPLICATION

(11) **EP 2 811 030 A2**
(43) Date of publication of application: **10.12.2014**
(21) Application number: 14183792.2
(22) Date of filing: 13.03.2009
(51) Int. Cl.: C12P 21/06, C07K 14/575, A61K 38/00

(54) **Improved beta Thymosin fragments**

(30) Priority: 17.03.2008 US 37207 P; 25.07.2008 US 83798 P
(62) Divisional of application: 09762984.4
(71) Applicant: Regenerx Biopharmaceuticals, Inc., Bethesda, MD 20814 (US)
(72) Inventor: The designation of the inventor has not yet been filed
(74) Representative: Klunker . Schmitt-Nilson . Hirsch

(57) **Abstract**

Peptide fragments having amino acid sequences corresponding to portions of a thymosin beta 4, a thymosin beta 10 and/or a thymosin beta 15 amino acid sequence are provided, as well as methods of treatment utilizing same.

## Description

### CROSS-REFERENCE TO RELATED APPLICATIONS

This application claims the benefit of U.S. Provisional Application Serial Number 61/083,798, filed July 25, 2008 and U.S. Provisional Application Serial No. 61/037,207, filed March 17, 2008, the contents of which are incorporated herein in their entirety by reference.

### BACKGROUND

### TECHNICAL FIELD

The present disclosure relates to the field of beta thymosin fragments.

### DESCRIPTION OF THE BACKGROUND ART

Thymosin β4 was initially identified as a protein that is up-regulated during endothelial cell migration and differentiation in vitro. Thymosin β4 (Tβ4) was originally isolated from the thymus and is a 43 amino acid, 4.9 kDa ubiquitous polypeptide identified in a variety of tissues. Several roles have been ascribed to this protein including a role in endothelial cell differentiation and migration, T cell differentiation, actin sequestration and vascularization.

The amino acid sequence of Tβ4 is disclosed in U.S. Patent No. 4,297,276, herein incorporated by reference. The gene encoding for Tβ4 was highly conserved during evolution. In fact, total homology exists between mice, rat and human Tβ4. Total homology is predicted to exist between the dog and human Tβ4 based on the analysis of a canine cDNA library.

Tβ4 has been found to be present in numerous tissue types in mammals and has also been implicated in a wide variety of cellular and physiological processes including inducing terminal deoxynucleotidyl transferase activity of bone marrow cells, stimulating secretion of hypothalamic luteinizing hormone releasing hormone and luteinizing hormone, inhibiting migration and enhancing antigen presentation of macrophages, and inducing phenotypic changes in T-cell lines *in vitro.*

There is a need in the art for active beta thymosin fragments.

### SUMMARY OF THE INVENTION

In accordance with one embodiment, a peptide fragment having an amino acid sequence corresponding to a portion of a thymosin beta 4, a thymosin beta 10 and/or a thymosin beta 15 amino acid sequence, comprises, consists essentially of, or consists of amino acid sequence H-Leu-Lys-Lys-Thr-Glu-Thr, H-Ser-Asp-Lys-Pro, Ser-Asp-Lys-Pro-Asp-Met-Ala-Glu-Ile-Glu-Lys-Phe-Asp-Lys-Ser, H-Leu-Lys-Lys-Thr-Glu-Thr-Gln, Glu-Lys-Asn-Pro-Leu-Pro-Ser-Lys-Glu-Thr, Ile-Glu-Gln-Glu-Lys-Gln-Ala-Gly-Glu-Ser, Met-Ala-Glu-Ile-Glu-Lys-Phe-Asp-Lys-Ser, Ser-Asp-Lys-Pro-Asp-Met-Ala-Glu-Ile-Glu-Lys-Phe-Asp-Lys-Ser-Lys-Leu-Lys-Lys-Thr-Glu-Thr, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, a methionine-containing variant of said fragment in which said methionine is oxidized or superoxidized, a variant of said fragment which normally is methionine-containing but which has an amino acid substituent substituted for at least one methionine of the normally methionine-containing fragment, an isolated R-enantiomer of said fragment, an isolated S-enantiomer of said fragment, or a combination thereof.

### DETAILED DESCRIPTION

Beta thymosin fragments in accordance with the present invention can be provided by any suitable method, such as by solid phase peptide synthesis, one example of which is disclosed in U.S. Patent No. 5,512,656.

Many Tβ4 isoforms have been identified and have about 70%, or about 75%, or about 80% or more homology to the known amino acid sequence of Tβ4. Such isoforms include, for example, Tβ4^{ala}, Tβ9, Tβ10, Tβ11, Tβ12, Tβ13, Tβ14 and Tβ15. Similar to Tβ4, the Tβ10 and Tβ15 isoforms have been shown to sequester actin. Tβ4, and many of its isoforms share an amino acid sequence, LKKTET or LKKTNT, that appears to be involved in mediating actin sequestration or binding.

According to one embodiment, a peptide fragment is provided, having an amino acid sequence corresponding to a portion of a thymosin beta 4, a thymosin beta 10 and/or a thymosin beta 15 amino acid sequence, said fragment comprising, consisting essentially of, or consisting of amino acid sequence H-Leu-Lys-Lys-Thr-Glu-Thr, H-Ser-Asp-Lys-Pro, Ser-Asp-Lys-Pro-Asp-Met-Ala-Glu-Ile-Glu-Lys-Phe-Asp-Lys-Ser, H-Leu-Lys-Lys-Thr-Glu-Thr-Gln, Glu-Lys-Asn-Pro-Leu-Pro-Ser-Lys-Glu-Thr, Ile-Glu-Gln-Glu-Lys-Gln-Ala-Gly-Glu-Ser, Met-Ala-Glu-Ile-Glu-Lys-Phe-Asp-Lys-Ser, Ser-Asp-Lys-Pro-Asp-Met-Ala-Glu-Ile-Glu-Lys-Phe-Asp-Lys-Ser-Lys-Leu-Lys-Lys-Thr-Glu-Thr, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, a methionine-containing variant of said fragment in which said methionine is oxidized or superoxidized, a variant of said fragment which normally is methionine-containing but which has an amino acid substituent substituted for at least one methionine of the normally methionine-containing fragment, an isolated R-enantiomer of said fragment, an isolated S-enantiomer of said fragment, or a combination thereof. A normally methionine-containing variant of said fragment may correspond to a methionine-containing fragment, but has an amino acid substituent substituted for at least one methionine of the fragment. Whenever herein amino acid Met is identified, it is to be understood that said Met may be substituted with amino acid AAA, wherein AAA may comprise leucine, valine, isoleucine, alanine, phenylalanine, proline or the like, substituted for said methionine.

Many beta thymosin peptides and fragments thereof include in their amino acid sequences the amino acid methionine, which is subject to oxidation *in vivo* and *in vitro.* In many of the known beta thymosins, methionine is present at position 6.

The oxidation of amino acid, methionine (C₅H₁₁NO₂S), to methionine sulfoxide (C₅H₁₁NO₃S), or otherwise, represents a major degradation pathway of methionine-containing beta thymosins such as Tβ4 and fragments thereof.

Exemplary beta thymosins containing methionine at position 6 include Tβ4, Tβ4^{ala}, Tβ4^{xen}, Tβ9^{met}, Tβ10 and Tβ13.

In preferred embodiments, the amino acid substituted for methionine is neutral, non-polar, hydrophobic and/or non-oxidizing.

The compositions have advantages in greater stability than methionine-containing beta thymosins, while possessing activity substantially the same as, or different from the corresponding beta thymosin fragment.

In preferred embodiments, the amino acid being substituted for methionine inhibits oxidation of the beta thymosin fragment, and most preferably, the biological activity of the substituted beta thymosin fragment is substantially the same as that of the corresponding methionine-containing beta thymosin fragment.

Replacement of methionine in a methionine-containing beta thymosin peptide fragment may result in a change in the stability profile of the peptide, and/or unexpectantly new or unchanged properties of the peptide fragment.

As non-limiting examples, the amino acid to be substituted for methionine in the methionine-containing beta thymosin fragment is valine, isoleucine, alanine, phenylalanine, proline or leucine.

In accordance with one embodiment, the amino acid to be substitute for methionine in the methionine-containing beta thymosin fragment is other than leucine. In certain embodiments, the amino acid to be substituted for methionine in the methionine-containing beta thymosin is valine, isoleucine, alanine, phenylalanine or proline.

In accordance with one embodiment, the preferred amino acid to be substituted for methionine is valine or isoleucine.

In accordance with another embodiment, the preferred amino acid to be substituted for methionine is alanine.

In accordance with a still further embodiment, the preferred amino acid to be substituted for methionine is valine.

Beta thymosin fragments and variants thereof in accordance with the present invention can be provided by any suitable method, such as by solid phase peptide synthesis, one example of which is disclosed in U.S. Patent No. 5,512,656.

PCT publication number WO 2006/076523 A1 discloses test results showing activity of Met-substituted beta thymosin peptides.

The invention also is applicable to combinations of fragments disclosed herein, which may be formed by admixing two or more different fragments (a physical mixing), or by chemically linking two or more different fragments using any suitable linkage method.

According to one embodiment, the fragment comprises amino acid sequence Ac-Leu-Lys-Lys-Thr-Glu-Thr-OH.

According to one embodiment, the fragment comprises amino acid sequence H-Ser-Asp-Lys-Pro-OH.

According to one embodiment, the fragment comprises amino acid sequence H-Ser-Asp-Lys-Pro-Asp-Met-Ala-Glu-Ile-Glu-Lys-Phe-Asp-Lys-Ser-OH.

According to one embodiment, the fragment comprises amino acid sequence Ac-Ser-Asp-Lys-Pro-Asp-Met-Ala-Glu-Ile-Glu-Lys-Phe-Asp-Lys-Ser-OH.

According to one embodiment, the fragment comprises amino acid sequence H-Leu-Lys-Lys-Thr-Glu-Thr-Gln-OH.

According to one embodiment, the fragment comprises amino acid sequence H-Glu-Lys-Asn-Pro-Leu-Pro-Ser-Lys-Glu-Thr-OH.

According to one embodiment, the fragment comprises amino acid sequence Ac-Glu-Lys-Asn-Pro-Leu-Pro-Ser-Lys-Glu-Thr-OH.

According to one embodiment, the fragment comprises amino acid sequence H-Ile-Glu-Gln-Glu-Lys-Gln-Ala-Gly-Glu-Ser-OH.

According to one embodiment, the fragment comprises amino acid sequence Ac-Ile-Glu-Gln-Glu-Lys-Gln-Ala-Gly-Glu-Ser-OH.

According to one embodiment, the fragment comprises amino acid sequence H-Met-Ala-Glu-Ile-Glu-Lys-Phe-Asp-Lys-Ser-OH.According to one embodiment, the fragment comprises amino acid sequence Ac-Met-Ala-Glu-Ile-Glu-Lys-Phe-Asp-Lys-Ser-OH.

According to one embodiment, the fragment comprises amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH.

According to one embodiment, the fragment comprises amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH.

According to one embodiment, the fragment comprises amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH.

According to one embodiment, the fragment comprises amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH.

According to one embodiment, the fragment comprises amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH.

According to one embodiment, the fragment comprises amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH.

According to one embodiment, the fragment comprises amino acid sequence Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH.

According to one embodiment, the fragment comprises amino acid sequence H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Leu-Lys-Lys-Thr-Glu-Thr-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Leu-Lys-Lys-Thr-Glu-Thr-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro-Asp-Met-Ala-Glu-Ile-Glu-Lys-Phe-Asp-Lys-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ser-Asp-Lys-Pro-Asp-Met-Ala-Glu-Ile-Glu-Lys-Phe-Asp-Lys-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Leu-Lys-Lys-Thr-Glu-Thr-Gln-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Glu-Lys-Asn-Pro-Leu-Pro-Ser-Lys-Glu-Thr-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Glu-Lys-Asn-Pro-Leu-Pro-Ser-Lys-Glu-Thr-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Ile-Glu-Gln-Glu-Lys-Gln-Ala-Gly-Glu-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ile-Glu-Gln-Glu-Lys-Gln-Ala-Gly-Glu-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Met-Ala-Glu-Ile-Glu-Lys-Phe-Asp-Lys-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Met-Ala-Glu-Ile-Glu-Lys-Phe-Asp-Lys-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence H-Leu-Lys-Lys-Thr-Glu-Thr.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Leu-Lys-Lys-Thr-Glu-Thr.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro-Asp-Met-Ala-Glu-Ile-Glu-Lys-Phe-Asp-Lys-Ser.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ser-Asp-Lys-Pro-Asp-Met-Ala-Glu-Ile-Glu-Lys-Phe-Asp-Lys-Ser.

According to one embodiment, a peptide fragment is provided, having an amino acid sequence corresponding to a portion of a thymosin beta 4 amino acid sequence, said fragment comprising, consisting essentially of, or consisting of amino acid sequence H-Leu-Lys-Lys-Thr-Glu-Thr-Gln.

According to one embodiment, a peptide fragment is provided, having an amino acid sequence corresponding to a portion of a thymosin beta 4 amino acid sequence, said fragment comprising, consisting essentially of, or consisting of amino acid sequence H-Glu-Lys-Asn-Pro-Leu-Pro-Ser-Lys-Glu-Thr.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Glu-Lys-Asn-Pro-Leu-Pro-Ser-Lys-Glu-Thr.

According to one embodiment, a peptide fragment is provided, having an amino acid sequence corresponding to a portion of a thymosin beta 4 amino acid sequence, said fragment comprising, consisting essentially of, or consisting of amino acid sequence H-Ile-Glu-Gln-Glu-Lys-Gln-Ala-Gly-Glu-Ser.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ile-Glu-Gln-Glu-Lys-Gln-Ala-Gly-Glu-Ser.

According to one embodiment, a peptide fragment is provided, having an amino acid sequence corresponding to a portion of a thymosin beta 4 amino acid sequence, said fragment comprising, consisting essentially of, or consisting of amino acid sequence H-Met-Ala-Glu-Ile-Glu-Lys-Phe-Asp-Lys-Ser.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Met-Ala-Glu-Ile-Glu-Lys-Phe-Asp-Lys-Ser.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln.

According to one embodiment, a peptide fragment is provided, having an amino acid sequence corresponding to a portion of a thymosin beta 4 amino acid sequence, said fragment comprising, consisting essentially of, or consisting of amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys.

According to one embodiment, a peptide fragment is provided, having an amino acid sequence corresponding to a portion of a thymosin beta 4 amino acid sequence, said fragment comprising, consisting essentially of, or consisting of amino acid sequence H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser.

According to one embodiment, a peptide fragment is provided, having an amino acid sequence corresponding to a portion of a thymosin beta 4 amino acid sequence, said fragment comprising, consisting essentially of, or consisting of amino acid sequence Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser.

According to one embodiment, a peptide fragment is provided, having an amino acid sequence corresponding to a portion of a thymosin beta 4 amino acid sequence, said fragment comprising, consisting essentially of, or consisting of amino acid sequence Leu-Lys-Lys-Thr-Glu-Thr-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ser-Asp-Lys-Pro-Asp-Met-Ala-Glu-Ile-Glu-Lys-Phe-Asp-Lys-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence Leu-Lys-Lys-Thr-Glu-Thr-Gln-OH.

According to one embodiment, a peptide fragment is provided, having an amino acid sequence corresponding to a portion of a thymosin beta 4 amino acid sequence, said fragment comprising, consisting essentially of, or consisting of amino acid sequence Glu-Lys-Asn-Pro-Leu-Pro-Ser-Lys-Glu-Thr-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ile-Glu-Gln-Glu-Lys-Gln-Ala-Gly-Glu-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence Met-Ala-Glu-Ile-Glu-Lys-Phe-Asp-Lys-Ser-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ser-Asp-Lys-Pro-Asp-Met-Ala-Glu-Ile-Glu-Lys-Phe-Asp-Lys-Ser-Lys-Leu-Lys-Lys-Thr-Glu-Thr-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH.

According to one embodiment, a peptide fragment is provided, having an amino acid sequence corresponding to a portion of a thymosin beta 4 amino acid sequence, said fragment comprising, consisting essentially of, or consisting of amino acid sequence Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH.

According to one embodiment, a peptide fragment is provided, having an amino acid sequence corresponding to a portion of a thymosin beta 4 amino acid sequence, said fragment comprising, consisting essentially of, or consisting of amino acid sequence Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH.

According to one embodiment, the invention comprises a method of at least one of suppressing inflammation in tissue of a subject, stimulating cell migration in tissue of a subject, protecting tissue from cytotoxicity in tissue of a subject, inhibiting apoptosis in tissue of a subject, stimulating collagen in tissue of a subject, inhibiting collagen in tissue of a subject, stimulating collagen IV in tissue of a subject, stimulating elastin in tissue of a subject, inhibiting the activation of NFkB and its translocation in tissue of a subject, promoting neurite outgrowth, promoting neuron survival, stimulating production of L1, inhibiting tissue damage caused by ultraviolet (UV) radiation, protecting tissue from ultraviolet (UV) radiation damage, inhibiting IKBa phosphorylation in tissue of a subject, or restoring impaired T-lymphocyte blastogenic response comprising administering to said subject a peptide fragment having an amino acid sequence corresponding to a portion of a thymosin beta 4 amino acid sequence, said fragment comprising, consisting essentially of, or consisting of amino acid sequence H-Leu-Lys-Lys-Thr-Glu-Thr-OH, Ac-Leu-Lys-Lys-Thr-Glu-Thr-OH, H-Ser-Asp-Lys-Pro-OH, H-Ser-Asp-Lys-Pro-Asp-Met-Ala-Glu-Ile-Glu-Lys-Phe-Asp-Lys-Ser-OH, Ac-Ser-Asp-Lys-Pro-Asp-Met-Ala-Glu-Ile-Glu-Lys-Phe-Asp-Lys-Ser-OH, H-Leu-Lys-Lys-Thr-Glu-Thr-Gln-OH, Ac-Leu-Lys-Lys-Thr-Glu-Thr-Gln-OH, H-Glu-Lys-Asn-Pro-Leu-Pro-Ser-Lys-Glu-Thr-OH, Ac-Glu-Lys-Asn-Pro-Leu-Pro-Ser-Lys-Glu-Thr-OH, H-Ile-Glu-Gln-Glu-Lys-Gln-Ala-Gly-Glu-Ser-OH, Ac-Ile-Glu-Gln-Glu-Lys-Gln-Ala-Gly-Glu-Ser-OH, H-Met-Ala-Glu-Ile-Glu-Lys-Phe-Asp-Lys-Ser-OH, Ac-Met-Ala-Glu-Ile-Glu-Lys-Phe-Asp-Lys-Ser-OH, H-Leu-Lys-Lys-Thr-Glu-Thr, Ac-Leu-Lys-Lys-Thr-Glu-Thr, H-Ser-Asp-Lys-Pro, H-Ser-Asp-Lys-Pro-Asp-Met-Ala-Glu-Ile-Glu-Lys-Phe-Asp-Lys-Ser, Ac-Ser-Asp-Lys-Pro-Asp-Met-Ala-Glu-Ile-Glu-Lys-Phe-Asp-Lys-Ser, H-Leu-Lys-Lys-Thr-Glu-Thr-Gln, Ac-Leu-Lys-Lys-Thr-Glu-Thr-Gln, H-Glu-Lys-Asn-Pro-Leu-Pro-Ser-Lys-Glu-Thr, Ac-Glu-Lys-Asn-Pro-Leu-Pro-Ser-Lys-Glu-Thr, H-Ile-Glu-Gln-Glu-Lys-Gln-Ala-Gly-Glu-Ser, Ac-Ile-Glu-Gln-Glu-Lys-Gln-Ala-Gly-Glu-Ser, H-Met-Ala-Glu-Ile-Glu-Lys-Phe-Asp-Lys-Ser, Ac-Met-Ala-Glu-Ile-Glu-Lys-Phe-Asp-Lys-Ser, Leu-Lys-Lys-Thr-Glu-Thr-OH, Ser-Asp-Lys-Pro-Asp-Met-Ala-Glu-Ile-Glu-Lys-Phe-Asp-Lys-Ser-OH, Leu-Lys-Lys-Thr-Glu-Thr-Gln-OH, Glu-Lys-Asn-Pro-Leu-Pro-Ser-Lys-Glu-Thr-OH, Ile-Glu-Gln-Glu-Lys-Gln-Ala-Gly-Glu-Ser-OH, Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH , a methionine-containing variant of said fragment in which said methionine is oxidized (sulfoxide) or superoxidized (sulfone), a variant of said fragment which normally is methionine-containing but which has an amino acid substituent substituted for at least one methionine of the normally methionine-containing fragment, an isolated R-enantiomer of said fragment, an isolated S-enantiomer of said fragment, or a combination thereof.

In one embodiment, the disclosure provides a method of treatment for treating, preventing, inhibiting or reducing disease, damage, injury and/or wounding of a subject, or of tissue of a subject, by administering an effective amount of a composition which contains a peptide as described herein or a combination of peptides described herein. The administering may be directly or systemically. Examples of direct administration include, for example, contacting tissue, by direct application, intrathecal injection or inhalation, with a carrier comprising a solution, lotion, salve, gel, cream, paste, spray, suspension, colloid, colloidal suspension, mix of nanoparticles, aerosol droplets, dispersion, emulsion, hydrogel, ointment, or oil including a peptide as described herein. Systemic administration includes, for example, oral, intravenous, intraperitoneal, subcutaneous, intramuscular injections of a composition containing a peptide as described herein, in a pharmaceutically acceptable carrier such as water for injection or protective chitosan nanoparticle bundles. The subject preferably is mammalian, most preferably human.

Compositions, as described herein, may be administered in any suitable effective amount. For example, a composition as described herein may be administered in dosages within the range of about 0.0001-5,000,000 micrograms, more preferably in amounts within the range of about 0.01-50,000 micrograms, most preferably within the range of about 1-500 micrograms.

A composition as described herein can be administered daily, every other day, every other week, every other month, etc., with a single application or multiple applications per day of administration, such as applications 2, 3, 4 or more times per day of administration.

The disclosure also includes a pharmaceutical or cosmetic composition comprising a therapeutically effective amount of a composition as described herein in a pharmaceutically or cosmetically acceptable carrier. Such carriers include any suitable carrier, including those listed herein.

The approaches described herein involve various routes of administration or delivery of a composition as described herein, including any conventional administration techniques (for example, but not limited to, direct administration, local injection, inhalation, or systemic administration), to a subject. The methods and compositions using or containing a composition as described herein may be formulated into pharmaceutical or cosmetic compositions by admixture with pharmaceutically acceptable or cosmetically non-toxic excipients, additives or carriers or by incorporation or linkage of a composition to a carrier or chaperone molecule to allow for targeted delivery of a composition described herein to a preferred site in the body of a mammal or preferably man.

### EXAMPLES

In the examples herein, the tested fragments are identified as follows:

| **SEQ ID NO** | | |
|---|---|---|
| 1 | Fragment 1a | H-Leu-Lys-Lys-Thr-Glu-Thr-OH |
| 12 | Fragment 1b | Ac-Leu-Lys-Lys-Thr-Glu-Thr-OH |
| 2 | Fragment 2a | H-Ser-Asp-Lys-Pro-OH |
| 13 | Fragment 2b | Ac-Ser-Asp-Lys-Pro-OH |
| 3 | Fragment 3a | H-Ser-Asp-Lys-Pro-Asp-Met-Ala-Glu-Ile-Glu-Lys- Phe-Asp-Lys-Ser-OH |
| 14 | Fragment 3b | Ac-Ser-Asp-Lys-Pro-Asp-Met-Ala-Glu-Ile-Glu-Lys- Phe-Asp-Lys-Ser-OH |
| 4 | Fragment 4a | H-Leu-Lys-Lys-Thr-Glu-Thr-Gln-OH |
| 15 | Fragment 4b | Ac-Leu-Lys-Lys-Thr-Glu-Thr-Gln-OH |
| 5 | Fragment 5a | H-Glu-Lys-Asn-Pro-Leu-Pro-Ser-Lys-Glu-Thr-OH |
| 16 | Fragment 5b | Ac-Glu-Lys-Asn-Pro-Leu-Pro-Ser-Lys-Glu-Thr-OH |
| 6 | Fragment 6a | H-Ile-Glu-Gln-Glu-Lys-Gln-Ala-Gly-Glu-Ser-OH |
| 17 | Fragment 6b | Ac-Ile-Glu-Gln-Glu-Lys-Gln-Ala-Gly-Glu-Ser-OH |
| 7 | Fragment 7a | H-Met-Ala-Glu-Ile-Glu-Lys-Phe-Asp-Lys-Ser-OH |
| 18 | Fragment 7b | Ac-Met-Ala-Glu-Ile-Glu-Lys-Phe-Asp-Lys-Ser-OH |
| 8 | Fraction Aa: | H-Ser-Asp-Lys-Pro-Asp-Met-Ala-Glu-Ile-Glu-Lys- Phe-Asp-Lys-Ser-Lys-Leu-Lys-Lys-Thr-Glu-Thr- OH. |
| 19 | Fraction Ab: | Ac-Ser-Asp-Lys-Pro-Asp-Met-Ala-Glu-Ile-Glu-Lys- Phe-Asp-Lys-Ser-Lys-Leu-Lys-Lys-Thr-Glu-Thr- OH. |
| 9 | Fraction Ba: | H-Ser-Asp-Lys-Pro-Asp-Met-Ala-Glu-Ile-Glu-Lys- Phe-Asp-Lys-Ser-Lys-Leu-Lys-Lys-Thr-Glu-Thr- Gln-OH. |
| 20 | Fraction Bb: | Ac-Ser-Asp-Lys-Pro-Asp-Met-Ala-Glu-Ile-Glu-Lys- Phe-Asp-Lys-Ser-Lys-Leu-Lys-Lys-Thr-Glu-Thr- Gln-OH. |
| 10 | Fraction Ca: | H-Ser-Asp-Lys-Pro-Asp-Met-Ala-Glu-Ile-Glu-Lys- Phe-Asp-Lys-Ser-Lys-Leu-Lys-Lys-Thr-Glu-Thr- Gln-Glu-Lys-OH. |
| 21 | Fraction Cb: | Ac-Ser-Asp-Lys-Pro-Asp-Met-Ala-Glu-Ile-Glu-Lys- Phe-Asp-Lys-Ser-Lys-Leu-Lys-Lys-Thr-Glu-Thr- Gln-Glu-Lys-OH. |
| 11 | Fraction Da: | H-Leu-Lys-Lys-Thr-Glu-Thr-Gln-Glu-Lys-Asn-Pro- Leu-Pro-Ser-Lys-Glu-Thr-Ile-Glu-Gln-Glu-Lys- Gln-Ala-Gly-Glu-Ser-OH. |
| 22 | Fraction Db: | Ac-Leu-Lys-Lys-Thr-Glu-Thr-Gln-Glu-Lys-Asn- Pro-Leu-Pro-Ser-Lys-Glu-Thr-Ile-Glu-Gln-Glu- Lys-Gln-Ala-Gly-Glu-Ser-OH. |

### LDH Cytotoxicity Assay

- Primary passaged human dermal fibroblasts (Fb, Cascade Biologics, OR) were cultured to 80% confluence
- 2 ml chlorhexidene digluconate (CHX, 0.002%) in basal medium ± 1 µg/ml Tb4 or peptides was added to Fb for 1, 2, 3, or 4 hours
- 100 µl culture medium (n = 4) was added to 100 µl LDH reagent and incubated at room temperature for 20 minutes
- OD500nm was read and averages ± SEM were calculated
- Data was analyzed with probability of *p* ≤ 0.05

Tb4 and peptide 3A and 3B protect Fb against the cytotoxic effects of 0.002% CHX after a 2 hour exposure

Tb4 and peptide 3A protect Fb against the cytotoxic effects of 0.002% CHX after 1, 2, or 3 hours exposure

Peptide 3A protects Fb against the cytotoxic effects of 0.002% and 0.005% CHX after 2 hours exposure

Dose Response Analysis of Peptide 3A
- Human dermal fibroblasts (Fb, Cascade Biologics, OR) were cultured to 80% confluence
- 2 ml CHX (0.002%) in basal medium ± 1 µg/ml Tb4 or 1.0, 0.1, 0.01, or 0.001 µg/ml peptide 3A was added to Fb for 1, 2, 3, or 4 hours
- 100 µl culture medium (n = 4) was added to 100 µl LDH reagent and incubated at room temperature for 20 minutes
- OD500nm was read and averages ± SEM were calculated
- Data was analyzed with probability of *p* ≤ 0.05

1.0 µg/ml peptide 3A protects Fb against the cytotoxic effects of 0.002% CHX after 2 hours

Ability of Peptide 3A to Protect Against Other Cytotoxic Agents
- Human dermal fibroblasts (Fb, Cascade Biologics, OR) were cultured to 80% confluence
- 2 ml 10% ethanol, 1% hydrogen peroxide, or 0.01% benzalkonium chloride in basal medium ± 1 µg/ml Tb4 or 1.0, 0.1, 0.01, or 0.001 µg/ml peptide 3A was added to Fb for 1, 2, 3, or 4 hours
- 100 µl culture medium (n = 4) was added to 100 µl LDH reagent and incubated at room temperature for 20 minutes
- OD500nm was read and averages ± SEM were calculated
- Data was analyzed with probability of *p* ≤ 0.05

Peptide 3A does not protect Fb against the cytotoxic effects of 10% ethanol

Peptide 3A protects Fb against the cytotoxic effects of 1% hydrogen peroxide

Peptide 3A protects Fb against the cytotoxic effects of 0.01% benzalkonium chloride

### Cellular Mechanisms of Cytotoxicity

Chlorhexidine: CHX can release iron from ferritin, an iron storage protein, in a dose-dependent manner which might be related to mitochondrial permeability transition

Ethanol: Some reports indicate that iron is involved in ethanol-induced cytotoxicity

Hydrogen Peroxide: The principal mechanism of H2O2 toxicity is thought to involve the generation of hydroxyl radicals through its interactions with Fe2+ ions

Benzalkonium chloride: The mechanisms of cytotoxicity of H2O2 and BAK appeared to differ

### Effect of Tb4 and Peptide 3A on CHX-induced apoptosis

- Human dermal Fb were cultured to 80% confluence
- Fb were treated with 0.002% CHX + 1 µg/ml Tb4 or peptide 3A, 4A or 6A for 1.5 hours
- APOPercentage "dye" was added to the Fb cultures at a 1:50 dilution for an additional 30 minutes
- Excess dye was washed from the cells, and digital images were captured. Cells dyed red were apoptotic
- Adobe Photoshop was used to calculate the number of red pixels in six digitized images for each treatment
- Statistical analysis was done using the *t*-test

Tb4 and peptide 3A protect Fb against apoptosis induced by exposure to 0.002% CHX for 2 hours

Peptide 3A protects Gingival Fb against cytotoxicity induced by 0.002% CHX

Tb4 and Peptide 3A protect gingival Fb against apoptosis induced by exposure to 0.002% CHX for 2 hours

### Summary of Cytoxicity and Apoptosis Studies

- Peptide 3A can inhibit chlorhexidine digluconate, hydrogen peroxide, and benzalkonium chloride-induced *cytotoxicity* in cultured human dermal fibroblasts for up to 4 hours exposure
- Peptide 3A can inhibit chlorhexidine digluconate-induced *apoptosis* in cultured human dermal fibroblasts
- Peptide 3A can inhibit chlorhexidine digluconate and Peridex (diluted to 0.002% CHX) induced *cytotoxicity* in cultured human gingival fibroblasts
- Peptide 3A can inhibit chlorhexidine digluconate-induced *apoptosis* in cultured human gingival fibroblasts

### Effect of Tb4 and peptides on collagen type IV secretion

- Transformed human corneal epithelial cells (HCET) were cultured to 80% confluence
- Cells were treated with 1 µg/ml Tb4 or peptides for 24 hours
- Culture supernatant samples (n = 3 per experiment, total of 4 experiments) were analyzed using a collagen type IV ELISA
- Data was analyzed using the *t*-test

Peptides 2A and 4A stimulate collagen type IV secretion in corneal epithelial cells

### Effect of Tb4 and peptides on collagen secretion

- Human dermal fibroblasts (Fb) were cultured to 80% confluence
- Cells were treated with 1 µg/ml Tb4 or peptides for 24, 48, or 72 hours. Cells were treated once/day.
- Culture supernatants (n = 3, experiments repeated 5 times) were assayed for collagen secretion using a colorimetric assay that detects collagen types I, II, III, IV and V, and possibly VII

Peptide 5B stimulates the secretion of collagens by human dermal fibroblasts after 3 days of treatment

Peptides inhibit the secretion of collagens by human dermal fibroblasts

### Effect of Tb4 and peptides on elastin secretion

- Human dermal fibroblasts (Fb) were cultured to 80% confluence
- Cells were treated with 1 µg/ml Tb4 or peptides for 24, 48, or 72 hours. Cells were treated once/day.
- Culture supernatants (n = 3, experiments repeated 6 times) were assayed for elastin secretion using a colorimetric assay
- Data was analyzed using the *t*-test

Tb4 and peptides 4A, 4B, and 7B stimulate the secretion of elastin by dermal Fb

### Summary of Collagen and Elastin Studies

- Peptides 2A and 4A stimulate collagen IV secretion by corneal epithelial cells
- No peptides stimulate collagen type I secretion by dermal fibroblasts
- Peptide 5B stimulates the secretion of "collagens" by dermal fibroblasts, but the type of collagen is unknown
- Peptide 4A stimulates the secretion of elastin by dermal fibroblasts

### Analysis of IL-8 Secretion

- Human corneal epithelial cells or dermal Fb were cultured to 80% confluence
- Cells were pre-treated with 1 µg/ml Tb4 or peptides for one hour
- Cells were stimulated with 10 ng/ml TNF-a in the presence or absence of Tb4 or peptides
- Culture medium was analyzed for secreted IL-8 by ELISA
   (n = 3, experiments repeated twice)
- Data was analyzed using the *t*-test

Tb4 and Peptide 1A suppress TNF-a-stimulated IL8 secretion in corneal epithelial cells

Tb4 and Peptides 2B and 3A suppress TNF-a-stimulated IL8 secretion in dermal fibroblasts

### Analysis of NFkB Nuclear Translocation

- Human corneal epithelial cells were cultured to 80% confluence
- Cells were pre-treated with 1 µg/ml Tb4 or peptide 4A or 6A for one hour
- Cells were stimulated with 10 ng/ml TNF-a in the presence or absence of Tb4 or peptides for 15 minutes
- Cells were fixed and labeled with antibodies to the p65 subunit of NFkB

### Analysis of Filamentous Actin Distribution

- Human corneal epithelial cells were cultured to 80% confluence
- Cells were pre-treated with Tb4 (1, 5, or 10 µg/ml) or peptide 4A or 6A (1 µg/ml) for one hour
- Cells were stimulated with 10 ng/ml TNF-a in the presence or absence of Tb4 or peptides for 1 hour
- Cells were fixed and labeled with FITC-phalloidin

### Effect of Tb4 and peptides on the NFkB signaling pathway

- Cell-free assay
- Prepared 1.0, 0.1, and 0.01 ng/ml solutions of Tb4 or peptides in kinase buffer supplied with kit
- Added 10 µl of the solutions to wells of a 96-well plate coated with IkBa
- IKKb (20 mU) was added to wells
- 90 µl kinase reaction buffer (containing ATP) was added to the wells
- ELISA to detect phosphorylated IkBa

Peptide 6B may have an effect on the NFkB signaling pathway by inhibiting IkBa phosphorylation.

In a further embodiment, Fragments 1 a, 1 b, 2a, 2b, 3a, 3b, 4a, 4b, 5a, 5b, 6a, 6b, 7a, and 7b are utilized to inhibit tissue damage caused by ultraviolet (UV) radiation and/or protect tissue from ultraviolet (UV) radiation damage, promote neurite outgrowth, promote neuron survival, and stimulate production of L1.

In one embodiment, Fragment Aa stimulates cell migration in tissue of a subject, protect tissue from cytotoxicity in tissue of a subject, inhibit apoptosis in tissue of a subject, inhibit collagen in tissue of a subject, stimulate collagen IV in tissue of a subject, stimulate elastin in tissue of a subject, inhibit NFkB translocation in tissue of a subject, inhibit tissue damage caused by ultraviolet (UV) radiation and/or protect tissue from ultraviolet (UV) radiation damage, promote neurite outgrowth, promote neuron survival, and stimulate production of L1.

In one embodiment, Fragment Ab stimulates cell migration in tissue of a subject, protect tissue from cytotoxicity in tissue of a subject, inhibit apoptosis in tissue of a subject, stimulate collagen in tissue of a subject, inhibit collagen in tissue of a subject, inhibit NFkB translocation in tissue of a subject, , inhibit tissue damage caused by ultraviolet (UV) radiation and/or protect tissue from ultraviolet (UV) radiation damage, promote neurite outgrowth, promote neuron survival, and stimulate production of L1.

In one embodiment, Fragment Ba stimulates cell migration in tissue of a subject, protect tissue from cytotoxicity in tissue of a subject, inhibit apoptosis in tissue of a subject, inhibit collagen in tissue of a subject, stimulate collagen IV in tissue of a subject, stimulate elastin in tissue of a subject, inhibit NFkB translocation in tissue of a subject, inhibit tissue damage caused by ultraviolet (UV) radiation and/or protect tissue from ultraviolet (UV) radiation damage, promote neurite outgrowth, promote neuron survival, and stimulate production of L1.

In one embodiment, Fragment Bb stimulates cell migration in tissue of a subject, protect tissue from cytotoxicity in tissue of a subject, inhibit apoptosis in tissue of a subject, stimulate collagen in tissue of a subject, inhibit collagen in tissue of a subject, inhibit NFkB translocation in tissue of a subject, promote neurite outgrowth, inhibit tissue damage caused by ultraviolet (UV) radiation and/or protect tissue from ultraviolet (UV) radiation damage, promote neuron survival, and stimulate production of L1.

In one embodiment, Fragment Ca stimulates cell migration in tissue of a subject, protect tissue from cytotoxicity in tissue of a subject, inhibit apoptosis in tissue of a subject, inhibit collagen in tissue of a subject, stimulate collagen IV in tissue of a subject, stimulate elastin in tissue of a subject, inhibit NFkB translocation in tissue of a subject, promote neurite outgrowth, inhibit tissue damage caused by ultraviolet (UV) radiation and/or protect tissue from ultraviolet (UV) radiation damage, promote neuron survival, and stimulate production of L1.

In one embodiment, Fragment Cb stimulates cell migration in tissue of a subject, protect tissue from cytotoxicity in tissue of a subject, inhibit apoptosis in tissue of a subject, stimulate collagen in tissue of a subject, inhibit collagen in tissue of a subject, inhibit NFkB translocation in tissue of a subject, inhibit tissue damage caused by ultraviolet (UV) radiation and/or protect tissue from ultraviolet (UV) radiation damage, promote neurite outgrowth, promote neuron survival, and stimulate production of L1.

In one embodiment, Fragment Da inhibits collagen in tissue of a subject, stimulate elastin in tissue of a subject, inhibit tissue damage caused by ultraviolet (UV) radiation and/or protect tissue from ultraviolet (UV) radiation damage, promote neurite outgrowth, promote neuron survival, stimulate production of L1, inhibit IKBa phosphorylation, and restore impaired T-lymphocyte blastogenic response.

In one embodiment, Fragment Db inhibits collagen in tissue of a subject, stimulate elastin in tissue of a subject, inhibit tissue damage caused by ultraviolet (UV) radiation and/or protect tissue from ultraviolet (UV) radiation damage, promote neurite outgrowth, promote neuron survival, stimulate production of L1,and inhibit IKBa phosphorylation and restore impaired T-lymphocyte blastogenic response..

**Summary of Peptide Uses**

| | **Tb4** | **1A** | **1B** | **2A** | **2B** | **3A** | **3B** | **4A** | **4B** | **5A** | **5B** | **6A** | **6B** | **7A** | **7B** | **Aa** | **Ab** | **Ba** | **Bb** | **Ca** | **Cb** | **Da** | **Db** |
|---|---|---|---|---|---|---|---|---|---|---|---|---|---|---|---|---|---|---|---|---|---|---|---|
| **SEQ ID NO.** | | **1** | **12** | **2** | **13** | **3** | **14** | **4** | **15** | **5** | **16** | **6** | **17** | **7** | **18** | **8** | **19** | **9** | **20** | **10** | **21** | **11** | **22** |
| **Suppresses inflammation** | **P** | **P** | | | **P** | **P** | | | | | | | | | | **U** | **U** | **U** | **U** | **U** | **U** | | |
| **Stimulates Migration** | **P** | | | | **P** | | | | **P** | **P** | | | | | | **U** | **U** | **U** | **U** | **U** | **U** | | |
| **Protects from cytotoxicity** | **P** | | | | | **P** | **P** | | | | | | | | | **U** | **U** | **U** | **U** | **U** | **U** | | |
| **Inhibits apoptosis** | **P** | | | | | **P** | | | | | | | | | | **U** | **U** | **U** | **U** | **U** | **U** | | |
| **Stimulates collagens** | | | | | | | | | | | **P** | | | | | | **U** | | **U** | | **U** | | |
| **Inhibits collagens** | | | **P** | **P** | | | **P** | | | | | **P** | **P** | **P** | **U** | **U** | **U** | **U** | **U** | **U** | **U** | **U** | **U** |
| **Stimulates collagen IV** | | | | **P** | | | | **P** | | | | | | | | **U** | | **U** | | **U** | | | |
| **Stimulates Elastin** | **P** | | | | | | | **P** | **P** | | | | | | **U** | **U** | **U** | **U** | **U** | **U** | **U** | **U** | **U** |
| **Inhibits NFkB translocation** | **P** | | | | | | | **P** | | | | | | | | **U** | | **U** | | **U** | | | |
| **Inhibits IkBa phosphorylation** | | | | | | | | | | | | | **P** | | | | | | | | | **U** | **U** |
| **Promotes neurite outgrowth** | **P** | **U** | **U** | **U** | **U** | **U** | **U** | **U** | **U** | **U** | **U** | **U** | **U** | **U** | **U** | **U** | **U** | **U** | **U** | **U** | **U** | **U** | **U** |
| **Promotes neuron survival** | **P** | **U** | **U** | **U** | **U** | **U** | **U** | **U** | **U** | **U** | **U** | **U** | **U** | **U** | **U** | **U** | **U** | **U** | **U** | **U** | **U** | **U** | **U** |
| **Stimulates L1 production** | **P** | **U** | **U** | **U** | **U** | **U** | **U** | **U** | **U** | **U** | **U** | **U** | **U** | **U** | **U** | **U** | **U** | **U** | **U** | **U** | **U** | **U** | **U** |
| **Inhibits tissue damage caused by UV radiation** | **P** | **U** | **U** | **U** | **U** | **U** | **U** | **U** | **U** | **U** | **U** | **U** | **U** | **U** | **U** | **U** | **U** | **U** | **U** | **U** | **U** | **U** | **U** |
| **Protects tissue from UV radiation damage** | **P** | **U** | **U** | **U** | **U** | **U** | **U** | **U** | **U** | **U** | **U** | **U** | **U** | **U** | **U** | **U** | **U** | **U** | **U** | **U** | **U** | **U** | **U** |
| **Restores impaired T-lymphocyte blastogenic response** | **P** | | | | | | | | | | | | | | | | | | | | | **U** | **U** |
| **P=active** | **U=useful** | | | | | | | | | | | | | | | | | | | | | | |

### Particular embodiments of the invention are indicated in the following numbered paragraphs:

**1.** A peptide fragment having an amino acid sequence corresponding to a portion of at least one of a thymosin beta 4, a thymosin beta 10 or a thymosin beta 15 amino acid sequence, said fragment comprising amino acid sequence H-Leu-Lys-Lys-Thr-Glu-Thr-OH, Ac-Leu-Lys-Lys-Thr-Glu-Thr-OH, H-Ser-Asp-Lys-Pro-OH, H-Ser-Asp-Lys-Pro-Asp-Met-Ala-Glu-Ile-Glu-Lys-Phe-Asp-Lys-Ser-OH, Ac-Ser-Asp-Lys-Pro-Asp-Met-Ala-Glu-Ile-Glu-Lys-Phe-Asp-Lys-Ser-OH, H-Leu-Lys-Lys-Thr-Glu-Thr-Gln-OH, Ac-Leu-Lys-Lys-Thr-Glu-Thr-Gln-OH, H-Glu-Lys-Asn-Pro-Leu-Pro-Ser-Lys-Glu-Thr-OH, Ac-Glu-Lys-Asn-Pro-Leu-Pro-Ser-Lys-Glu-Thr-OH, H-Ile-Glu-Gln-Glu-Lys-Gln-Ala-Gly-Glu-Ser-OH, Ac-Ile-Glu-Gln-Glu-Lys-Gln-Ala-Gly-Glu-Ser-OH, H-Met-Ala-Glu-Ile-Glu-Lys-Phe-Asp-Lys-Ser-OH, Ac-Met-Ala-Glu-Ile-Glu-Lys-Phe-Asp-Lys-Ser-OH, H-Leu-Lys-Lys-Thr-Glu-Thr, Ac-Leu-Lys-Lys-Thr-Glu-Thr, H-Ser-Asp-Lys-Pro, H-Ser-Asp-Lys-Pro-Asp-Met-Ala-Glu-Ile-Glu-Lys-Phe-Asp-Lys-Ser, Ac-Ser-Asp-Lys-Pro-Asp-Met-Ala-Glu-Ile-Glu-Lys-Phe-Asp-Lys-Ser, H-Leu-Lys-Lys-Thr-Glu-Thr-Gln, Ac-Leu-Lys-Lys-Thr-Glu-Thr-Gln, H-Glu-Lys-Asn-Pro-Leu-Pro-Ser-Lys-Glu-Thr, Ac-Glu-Lys-Asn-Pro-Leu-Pro-Ser-Lys-Glu-Thr, H-Ile-Glu-Gln-Glu-Lys-Gln-Ala-Gly-Glu-Ser, Ac-Ile-Glu-Gln-Glu-Lys-Gln-Ala-Gly-Glu-Ser, H-Met-Ala-Glu-Ile-Glu-Lys-Phe-Asp-Lys-Ser, Ac-Met-Ala-Glu-Ile-Glu-Lys-Phe-Asp-Lys-Ser, Leu-Lys-Lys-Thr-Glu-Thr-OH, Ser-Asp-Lys-Pro-Asp-Met-Ala-Glu-Ile-Glu-Lys-Phe-Asp-Lys-Ser-OH, Leu-Lys-Lys-Thr-Glu-Thr-Gln-OH, Glu-Lys-Asn-Pro-Leu-Pro-Ser-Lys-Glu-Thr-OH, Ile-Glu-Gln-Glu-Lys-Gln-Ala-Gly-Glu-Ser-OH, Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, a methionine-containing variant of said fragment in which said methionine is oxidized or superoxidized, a variant of said fragment which normally is methionine-containing but which has an amino acid substituent substituted for at least one methionine of the normally methionine-containing fragment, an isolated R-enantiomer of said fragment, an isolated S-enantiomer of said fragment, or a combination thereof.
**2.** The fragment of paragraph 1, having amino acid sequence H-Leu-Lys-Lys-Thr-Glu-Thr-OH.
**3.** The fragment of paragraph 1, having amino acid sequence H-Ser-Asp-Lys-Pro-OH.
**4.** The fragment of paragraph 1, having amino acid sequence H-Ser-Asp-Lys-Pro-Asp-Met-Ala-Glu-Ile-Glu-Lys-Phe-Asp-Lys-Ser-OH.
**5.** The fragment of paragraph 1, having amino acid sequence Ac-Ser-Asp-Lys-Pro-Asp-Met-Ala-Glu-Ile-Glu-Lys-Phe-Asp-Lys-Ser-OH.
**6.** The fragment of paragraph 1, having amino acid sequence H-Leu-Lys-Lys-Thr-Glu-Thr-Gln-OH.
**7.** The fragment of paragraph 1, having amino acid sequence H-Glu-Lys-Asn-Pro-Leu-Pro-Ser-Lys-Glu-Thr-OH.
**8.** The fragment of paragraph 1, having amino acid sequence Ac-Glu-Lys-Asn-Pro-Leu-Pro-Ser-Lys-Glu-Thr-OH.
**9.** The fragment of paragraph 1, having amino acid sequence H-Ile-Glu-Gln-Glu-Lys-Gln-Ala-Gly-Glu-Ser-OH.
**10.** The fragment of paragraph 1, having amino acid sequence Ac-Ile-Glu-Gln-Glu-Lys-Gln-Ala-Gly-Glu-Ser-OH.
**11.** The fragment of paragraph 1, having amino acid sequence H-Met-Ala-Glu-Ile-Glu-Lys-Phe-Asp-Lys-Ser-OH.
**12.** The fragment of paragraph 1, having amino acid sequence Ac-Met-Ala-Glu-Ile-Glu-Lys-Phe-Asp-Lys-Ser-OH.
**13.** The fragment of paragraph 1, having amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH.
**14.** The fragment of paragraph 1, having amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH .
**15.** The fragment of paragraph 1, having amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH.
**16.** The fragment of paragraph 1, having amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH.
**17.** The fragment of paragraph 1, having amino acid sequence H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH .
**18.** The fragment of paragraph 1, having amino acid sequence Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH H-Leu-Lys-Lys-Thr-Glu-Thr-Gln-OH.
**19.** The fragment of paragraph 1, having amino acid sequence H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH.
**20.** The fragment of paragraph 1, having amino acid sequence Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH.
**21.** The fragment of paragraph 1, wherein said fragment corresponds to a methionine-containing fragment, but has an amino acid substituent substituted for at least one methionine of the methionine, said substituent comprising leucine, valine, isoleucine, alanine, phenylalanine or proline, substituted for said methionine.
**22.** A method of at least one of suppressing inflammation in tissue of a subject, stimulating cell migration in tissue of a subject, protecting tissue from cytotoxicity in tissue of a subject, inhibiting apoptosis in tissue of a subject, stimulating collagen in tissue of a subject, inhibiting collagen in tissue of a subject, stimulating collagen IV in tissue of a subject, stimulating elastin in tissue of a subject, inhibiting NFkB translocation in tissue of a subject, inhibiting tissue damage caused by ultraviolet (UV) radiation, protecting tissue from ultraviolet (UV) radiation damage, promoting neurite outgrowth, promoting neuron survival, stimulating production of L1, inhibiting IKBa phosphorylation, or restoring impaired T-lymphocyte blastogenic response comprising administering to said subject a peptide fragment having an amino acid sequence corresponding to a portion of a thymosin beta 4, a thymosin beta 10 or a thymosin beta 15 amino acid sequence, said fragment comprising amino acid sequence H-Leu-Lys-Lys-Thr-Glu-Thr-OH, Ac-Leu-Lys-Lys-Thr-Glu-Thr-OH, H-Ser-Asp-Lys-Pro-OH, H-Ser-Asp-Lys-Pro-Asp-Met-Ala-Glu-Ile-Glu-Lys-Phe-Asp-Lys-Ser-OH, Ac-Ser-Asp-Lys-Pro-Asp-Met-Ala-Glu-Ile-Glu-Lys-Phe-Asp-Lys-Ser-OH, H-Leu-Lys-Lys-Thr-Glu-Thr-Gln-OH, Ac-Leu-Lys-Lys-Thr-Glu-Thr-Gln-OH, H-Glu-Lys-Asn-Pro-Leu-Pro-Ser-Lys-Glu-Thr-OH, Ac-Glu-Lys-Asn-Pro-Leu-Pro-Ser-Lys-Glu-Thr-OH, H-Ile-Glu-Gln-Glu-Lys-Gln-Ala-Gly-Glu-Ser-OH, Ac-Ile-Glu-Gln-Glu-Lys-Gln-Ala-Gly-Glu-Ser-OH, H-Met-Ala-Glu-Ile-Glu-Lys-Phe-Asp-Lys-Ser-OH, Ac-Met-Ala-Glu-Ile-Glu-Lys-Phe-Asp-Lys-Ser-OH, H-Leu-Lys-Lys-Thr-Glu-Thr, Ac-Leu-Lys-Lys-Thr-Glu-Thr, H-Ser-Asp-Lys-Pro, H-Ser-Asp-Lys-Pro-Asp-Met-Ala-Glu-Ile-Glu-Lys-Phe-Asp-Lys-Ser, Ac-Ser-Asp-Lys-Pro-Asp-Met-Ala-Glu-Ile-Glu-Lys-Phe-Asp-Lys-Ser, H-Leu-Lys-Lys-Thr-Glu-Thr-Gln, Ac-Leu-Lys-Lys-Thr-Glu-Thr-Gln, H-Glu-Lys-Asn-Pro-Leu-Pro-Ser-Lys-Glu-Thr, Ac-Glu-Lys-Asn-Pro-Leu-Pro-Ser-Lys-Glu-Thr, H-Ile-Glu-Gln-Glu-Lys-Gln-Ala-Gly-Glu-Ser, Ac-Ile-Glu-Gln-Glu-Lys-Gln-Ala-Gly-Glu-Ser, H-Met-Ala-Glu-Ile-Glu-Lys-Phe-Asp-Lys-Ser, Ac-Met-Ala-Glu-Ile-Glu-Lys-Phe-Asp-Lys-Ser, Leu-Lys-Lys-Thr-Glu-Thr-OH, Ser-Asp-Lys-Pro-Asp-Met-Ala-Glu-Ile-Glu-Lys-Phe-Asp-Lys-Ser-OH, Leu-Lys-Lys-Thr-Glu-Thr-Gln-OH, Glu-Lys-Asn-Pro-Leu-Pro-Ser-Lys-Glu-Thr-OH, Ile-Glu-Gln-Glu-Lys-Gln-Ala-Gly-Glu-Ser-OH, Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gin, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, a methionine-containing variant of said fragment in which said methionine is oxidized or superoxidized, a variant of said fragment which normally is methionine-containing but which has an amino acid substituent substituted for at least one methionine of the normally methionine-containing fragment, an isolated R-enantiomer of said fragment, an isolated S-enantiomer of said fragment, or a combination thereof.
**23.** The method of paragraph 22, wherein said at least one of inhibiting tissue damage caused by ultraviolet (UV) radiation or protecting tissue from ultraviolet (UV) radiation damage comprises administering a peptide fragment having an amino acid sequence comprising H-Leu-Lys-Lys-Thr-Glu-Thr-OH, Ac-Leu-Lys-Lys-Thr-Glu-Thr-OH, H-Ser-Asp-Lys-Pro-OH, H-Ser-Asp-Lys-Pro-Asp-Met-Ala-Glu-Ile-Glu-Lys-Phe-Asp-Lys-Ser-OH, Ac-Ser-Asp-Lys-Pro-Asp-Met-Ala-Glu-Ile-Glu-Lys-Phe-Asp-Lys-Ser-OH, H-Leu-Lys-Lys-Thr-Glu-Thr-Gln-OH, Ac-Leu-Lys-Lys-Thr-Glu-Thr-Gln-OH, H-Glu-Lys-Asn-Pro-Leu-Pro-Ser-Lys-Glu-Thr-OH, Ac-Glu-Lys-Asn-Pro-Leu-Pro-Ser-Lys-Glu-Thr-OH, H-Ile-Glu-Gln-Glu-Lys-Gln-Ala-Gly-Glu-Ser-OH, Ac-Ile-Glu-Gln-Glu-Lys-Gln-Ala-Gly-Glu-Ser-OH, H-Met-Ala-Glu-Ile-Glu-Lys-Phe-Asp-Lys-Ser-OH, Ac-Met-Ala-Glu-Ile-Glu-Lys-Phe-Asp-Lys-Ser-OH, H-Leu-Lys-Lys-Thr-Glu-Thr, Ac-Leu-Lys-Lys-Thr-Glu-Thr, H-Ser-Asp-Lys-Pro, H-Ser-Asp-Lys-Pro-Asp-Met-Ala-Glu-Ile-Glu-Lys-Phe-Asp-Lys-Ser, Ac-Ser-Asp-Lys-Pro-Asp-Met-Ala-Glu-Ile-Glu-Lys-Phe-Asp-Lys-Ser, H-Leu-Lys-Lys-Thr-Glu-Thr-Gln, Ac-Leu-Lys-Lys-Thr-Glu-Thr-Gln, H-Glu-Lys-Asn-Pro-Leu-Pro-Ser-Lys-Glu-Thr, Ac-Glu-Lys-Asn-Pro-Leu-Pro-Ser-Lys-Glu-Thr, H-Ile-Glu-Gln-Glu-Lys-Gln-Ala-Gly-Glu-Ser, Ac-Ile-Glu-Gln-Glu-Lys-Gln-Ala-Gly-Glu-Ser, H-Met-Ala-Glu-Ile-Glu-Lys-Phe-Asp-Lys-Ser, Ac-Met-Ala-Glu-Ile-Glu-Lys-Phe-Asp-Lys-Ser, Leu-Lys-Lys-Thr-Glu-Thr-OH, Ser-Asp-Lys-Pro-Asp-Met-Ala-Glu-Ile-Glu-Lys-Phe-Asp-Lys-Ser-OH, Leu-Lys-Lys-Thr-Glu-Thr-Gln-OH, Glu-Lys-Asn-Pro-Leu-Pro-Ser-Lys-Glu-Thr-OH, Ile-Glu-Gln-Glu-Lys-Gln-Ala-Gly-Glu-Ser-OH, Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, or a combination thereof.
**24.** The method of paragraph 22, wherein said suppressing of inflammation in tissue of a subject comprises administering a peptide fragment having an amino acid sequence comprising H-Leu-Lys-Lys-Thr-Glu-Thr-OH, H-Ser-Asp-Lys-Pro-Asp-Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr, H-Ser-Asp-Lys-Pro-Asp-Met-Ala-Glu-Ile-Glu-Lys-Phe-Asp-Lys-Ser, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, or a combination thereof.
**25.** The method of paragraph 22, wherein said stimulating cell migration in tissue of a subject comprises administering a peptide fragment having an amino acid sequence comprising Ac-Leu-Lys-Lys-Thr-Glu-Thr-Gln-OH, H-Glu-Lys-Asn-Pro-Leu-Pro-Ser-Lys-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Leu-Lys-Lys-Thr-Glu-Thr-Gln-OH, H-Glu-Lys-Asn-Pro-Leu-Pro-Ser-Lys-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, or a combination thereof.
**26.** The method of paragraph 22, wherein said protecting tissue from cytotoxicity in tissue of a subject comprises administering a peptide fragment having an amino acid sequence comprising H-Ser-Asp-Lys-Pro-Asp-Met-Ala-Glu-Ile-Glu-Lys-Phe-Asp-Lys-Ser-OH, Ac-Ser-Asp-Lys-Pro-Asp-Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ser-Asp-Lys-Pro-Asp-Met-Ala-Glu-Ile-Glu-Lys-Phe-Asp-Lys-Ser-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Ser-Asp-Lys-Pro-Asp-Met-Ala-Glu-Ile-Glu-Lys-Phe-Asp-Lys-Ser, Ac-Ser-Asp-Lys-Pro-Asp-Met-Ala-Glu-Ile-Glu-Lys-Phe-Asp-Lys-Ser, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, or a combination thereof.
**27.** The method of paragraph 22, wherein said inhibiting apoptosis in tissue of a subject comprises administering a peptide fragment having an amino acid sequence comprising H-Ser-Asp-Lys-Pro-Asp-Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Ser-Asp-Lys-Pro-Asp-Met-Ala-Glu-Ile-Glu-Lys-Phe-Asp-Lys-Ser, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, or a combination thereof.
**28.** The method of paragraph 22, wherein said stimulating collagen in tissue of a subject comprises administering a peptide fragment having an amino acid sequence comprising Ac-Glu-Lys-Asn-Pro-Leu-Pro-Ser-Lys-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Glu-Lys-Asn-Pro-Leu-Pro-Ser-Lys-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, or a combination thereof.
**29.** The method of paragraph 22, wherein said inhibiting collagen in tissue of a subject comprises administering a peptide fragment having an amino acid sequence comprising Ac-Leu-Lys-Lys-Thr-Glu-Thr-OH, H-Ser-Asp-Lys-Pro-OH, Ac-Ser-Asp-Lys-Pro-Asp-Met-Ala-Glu-Ile-Glu-Lys-Phe-Asp-Lys-Ser-OH, H-Ile-Glu-Gln-Glu-Lys-Gln-Ala-Gly-Glu-Ser-OH, Ac-Ile-Glu-Gln-Glu-Lys-Gln-Ala-Gly-Glu-Ser-OH, H-Met-Ala-Glu-Ile-Glu-Lys-Phe-Asp-Lys-Ser-OH, Ac-Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ile-Glu-Gln-Glu-Lys-Gln-Ala-Gly-Glu-Ser, Met-Ala-Glu-Ile-Glu-Lys-Phe-Asp-Lys-Ser-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-OH, H-Ser-Asp-Lys-Pro, Ac-Ser-Asp-Lys-Pro-Asp-Met-Ala-Glu-Ile-Glu-Lys-Phe-Asp-Lys-Ser, H-Ile-Glu-Gln-Glu-Lys-Gin-Ala-Gly-Glu-Ser, Ac-Ile-Glu-Gln-Glu-Lys-Gln-Ala-Gly-Glu-Ser, H-Met-Ala-Glu-Ile-Glu-Lys-Phe-Asp-Lys-Ser, Ac-Met-Ala-Glu-Ile-Glu-Lys-Phe-Asp-Lys-Ser, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gin-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, or a combination thereof.
**30.** The method of paragraph 22, wherein said stimulating collagen IV in tissue of a subject comprises administering a peptide fragment having an amino acid sequence comprising H-Ser-Asp-Lys-Pro-OH, H-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Ser-Asp-Lys-Pro, H-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, or a combination thereof.
**31.** The method of paragraph 22, wherein said stimulating elastin in tissue of a subject comprises administering a peptide fragment having an amino acid sequence comprising H-Leu-Lys-Lys-Thr-Glu-Thr-Gln-OH, Ac-Leu-Lys-Lys-Thr-Glu-Thr-Gln-OH, Ac-Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, H-Leu-Lys-Lys-Thr-Glu-Thr-Gln, Ac-Leu-Lys-Lys-Thr-Glu-Thr-Gln, Ac-Met-Ala-Glu-Ile-Glu-Lys-Phe-Asp-Lys-Ser, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, or a combination thereof.
**32.** The method of paragraph 22, wherein said inhibiting NFkB translocation in tissue of a subject comprises administering a peptide fragment having an amino acid sequence comprising H-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, or a combination thereof.
**33.** The method of paragraph 22, wherein said promoting neurite outgrowth administering a peptide fragment having an amino acid sequence comprising H-Leu-Lys-Lys-Thr-Glu-Thr-OH, Ac-Leu-Lys-Lys-Thr-Glu-Thr-OH, H-Ser-Asp-Lys-Pro-OH, H-Ser-Asp-Lys-Pro-Asp-Met-Ala-Glu-Ile-Glu-Lys-Phe-Asp-Lys-Ser-OH, Ac-Ser-Asp-Lys-Pro-Asp-Met-Ala-Glu-Ile-Glu-Lys-Phe-Asp-Lys-Ser-OH, H-Leu-Lys-Lys-Thr-Glu-Thr-Gln-OH, Ac-Leu-Lys-Lys-Thr-Glu-Thr-Gln-OH, H-Glu-Lys-Asn-Pro-Leu-Pro-Ser-Lys-Glu-Thr-OH, Ac-Glu-Lys-Asn-Pro-Leu-Pro-Ser-Lys-Glu-Thr-OH, H-Ile-Glu-Gln-Glu-Lys-Gln-Ala-Gly-Glu-Ser-OH, Ac-Ile-Glu-Gln-Glu-Lys-Gln-Ala-Gly-Glu-Ser-OH, H-Met-Ala-Glu-Ile-Glu-Lys-Phe-Asp-Lys-Ser-OH, Ac-Met-Ala-Glu-Ile-Glu-Lys-Phe-Asp-Lys-Ser-OH, H-Leu-Lys-Lys-Thr-Glu-Thr, Ac-Leu-Lys-Lys-Thr-Glu-Thr, H-Ser-Asp-Lys-Pro, H-Ser-Asp-Lys-Pro-Asp-Met-Ala-Glu-Ile-Glu-Lys-Phe-Asp-Lys-Ser, Ac-Ser-Asp-Lys-Pro-Asp-Met-Ala-Glu-Ile-Glu-Lys-Phe-Asp-Lys-Ser, H-Leu-Lys-Lys-Thr-Glu-Thr-Gln, Ac-Leu-Lys-Lys-Thr-Glu-Thr-Gln, H-Glu-Lys-Asn-Pro-Leu-Pro-Ser-Lys-Glu-Thr, Ac-Glu-Lys-Asn-Pro-Leu-Pro-Ser-Lys-Glu-Thr, H-Ile-Glu-Gln-Glu-Lys-Gln-Ala-Gly-Glu-Ser, Ac-Ile-Glu-Gln-Glu-Lys-Gln-Ala-Gly-Glu-Ser, H-Met-Ala-Glu-Ile-Glu-Lys-Phe-Asp-Lys-Ser, Ac-Met-Ala-Glu-Ile-Glu-Lys-Phe-Asp-Lys-Ser, Leu-Lys-Lys-Thr-Glu-Thr-OH, Ser-Asp-Lys-Pro-Asp-Met-Ala-Glu-Ile-Glu-Lys-Phe-Asp-Lys-Ser-OH, Leu-Lys-Lys-Thr-Glu-Thr-Gln-OH, Glu-Lys-Asn-Pro-Leu-Pro-Ser-Lys-Glu-Thr-OH, Ile-Glu-Gln-Glu-Lys-Gln-Ala-Gly-Glu-Ser-OH, Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, or a combination thereof.
**34.** The method of paragraph 22, wherein said promoting neuron survival comprises administering a peptide fragment having an amino acid sequence H-Leu-Lys-Lys-Thr-Glu-Thr-OH, Ac-Leu-Lys-Lys-Thr-Glu-Thr-OH, H-Ser-Asp-Lys-Pro-OH, H-Ser-Asp-Lys-Pro-Asp-Met-Ala-Glu-Ile-Glu-Lys-Phe-Asp-Lys-Ser-OH, Ac-Ser-Asp-Lys-Pro-Asp-Met-Ala-Glu-Ile-Glu-Lys-Phe-Asp-Lys-Ser-OH, H-Leu-Lys-Lys-Thr-Glu-Thr-Gln-OH, Ac-Leu-Lys-Lys-Thr-Glu-Thr-Gln-OH, H-Glu-Lys-Asn-Pro-Leu-Pro-Ser-Lys-Glu-Thr-OH, Ac-Glu-Lys-Asn-Pro-Leu-Pro-Ser-Lys-Glu-Thr-OH, H-Ile-Glu-Gln-Glu-Lys-Gln-Ala-Gly-Glu-Ser-OH, Ac-Ile-Glu-Gln-Glu-Lys-Gln-Ala-Gly-Glu-Ser-OH, H-Met-Ala-Glu-Ile-Glu-Lys-Phe-Asp-Lys-Ser-OH, Ac-Met-Ala-Glu-Ile-Glu-Lys-Phe-Asp-Lys-Ser-OH, H-Leu-Lys-Lys-Thr-Glu-Thr, Ac-Leu-Lys-Lys-Thr-Glu-Thr, H-Ser-Asp-Lys-Pro, H-Ser-Asp-Lys-Pro-Asp-Met-Ala-Glu-Ile-Glu-Lys-Phe-Asp-Lys-Ser, Ac-Ser-Asp-Lys-Pro-Asp-Met-Ala-Glu-Ile-Glu-Lys-Phe-Asp-Lys-Ser, H-Leu-Lys-Lys-Thr-Glu-Thr-Gln, Ac-Leu-Lys-Lys-Thr-Glu-Thr-Gln, H-Glu-Lys-Asn-Pro-Leu-Pro-Ser-Lys-Glu-Thr, Ac-Glu-Lys-Asn-Pro-Leu-Pro-Ser-Lys-Glu-Thr, H-Ile-Glu-Gln-Glu-Lys-Gln-Ala-Gly-Glu-Ser, Ac-Ile-Glu-Gln-Glu-Lys-Gln-Ala-Gly-Glu-Ser, H-Met-Ala-Glu-Ile-Glu-Lys-Phe-Asp-Lys-Ser, Ac-Met-Ala-Glu-Ile-Glu-Lys-Phe-Asp-Lys-Ser, Leu-Lys-Lys-Thr-Glu-Thr-OH, Ser-Asp-Lys-Pro-Asp-Met-Ala-Glu-Ile-Glu-Lys-Phe-Asp-Lys-Ser-OH, Leu-Lys-Lys-Thr-Glu-Thr-Gln-OH, Glu-Lys-Asn-Pro-Leu-Pro-Ser-Lys-Glu-Thr-OH, Ile-Glu-Gln-Glu-Lys-Gln-Ala-Gly-Glu-Ser-OH, Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, or a combination thereof.
**35.** The method of paragraph 22, wherein said stimulating production of L1 comprises administering a peptide fragment having an amino acid sequence comprising H-Leu-Lys-Lys-Thr-Glu-Thr-OH, Ac-Leu-Lys-Lys-Thr-Glu-Thr-OH, H-Ser-Asp-Lys-Pro-OH, H-Ser-Asp-Lys-Pro-Asp-Met-Ala-Glu-Ile-Glu-Lys-Phe-Asp-Lys-Ser-OH, Ac-Ser-Asp-Lys-Pro-Asp-Met-Ala-Glu-Ile-Glu-Lys-Phe-Asp-Lys-Ser-OH, H-Leu-Lys-Lys-Thr-Glu-Thr-Gln-OH, Ac-Leu-Lys-Lys-Thr-Glu-Thr-Gln-OH, H-Glu-Lys-Asn-Pro-Leu-Pro-Ser-Lys-Glu-Thr-OH, Ac-Glu-Lys-Asn-Pro-Leu-Pro-Ser-Lys-Glu-Thr-OH, H-Ile-Glu-Gln-Glu-Lys-Gln-Ala-Gly-Glu-Ser-OH, Ac-Ile-Glu-Gln-Glu-Lys-Gln-Ala-Gly-Glu-Ser-OH, H-Met-Ala-Glu-Ile-Glu-Lys-Phe-Asp-Lys-Ser-OH, Ac-Met-Ala-Glu-Ile-Glu-Lys-Phe-Asp-Lys-Ser-OH, H-Leu-Lys-Lys-Thr-Glu-Thr, Ac-Leu-Lys-Lys-Thr-Glu-Thr, H-Ser-Asp-Lys-Pro, H-Ser-Asp-Lys-Pro-Asp-Met-Ala-Glu-Ile-Glu-Lys-Phe-Asp-Lys-Ser, Ac-Ser-Asp-Lys-Pro-Asp-Met-Ala-Glu-Ile-Glu-Lys-Phe-Asp-Lys-Ser, H-Leu-Lys-Lys-Thr-Glu-Thr-Gln, Ac-Leu-Lys-Lys-Thr-Glu-Thr-Gln, H-Glu-Lys-Asn-Pro-Leu-Pro-Ser-Lys-Glu-Thr, Ac-Glu-Lys-Asn-Pro-Leu-Pro-Ser-Lys-Glu-Thr, H-Ile-Glu-Gln-Glu-Lys-Gln-Ala-Gly-Glu-Ser, Ac-Ile-Glu-Gln-Glu-Lys-Gln-Ala-Gly-Glu-Ser, H-Met-Ala-Glu-Ile-Glu-Lys-Phe-Asp-Lys-Ser, Ac-Met-Ala-Glu-Ile-Glu-Lys-Phe-Asp-Lys-Ser, Leu-Lys-Lys-Thr-Glu-Thr-OH, Ser-Asp-Lys-Pro-Asp-Met-Ala-Glu-Ile-Glu-Lys-Phe-Asp-Lys-Ser-OH, Leu-Lys-Lys-Thr-Glu-Thr-Gln-OH, Glu-Lys-Asn-Pro-Leu-Pro-Ser-Lys-Glu-Thr-OH, Ile-Glu-Gln-Glu-Lys-Gln-Ala-Gly-Glu-Ser-OH, Met-Ala-Glu-Ile-Glu-Lys-Phe-Asp-Lys-Ser-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, H-Ser-Asp-Lys-Pro-Asp-Met-Ala-Glu-lle-Glu-Lys-Phe-Asp-Lys-Ser-Lys-Leu-Lys-Lys-Thr-Glu-Thr, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln, H-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, Ac-Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ser-Asp-Lys-Pro-Asp-Met-Ala-Glu-Ile-Glu-Lys-Phe-Asp-Lys-Ser-Lys-Leu-Lys-Lys-Thr-Glu-Thr-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-OH, Ser-Asp-Lys-Pro-Asp-Met-Ala-Glu-Ile-Glu-Lys-Phe-Asp-Lys-Ser-Lys-Leu-Lys-Lys-Thr-Glu-Thr-Gln-Glu-Lys-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, or a combination thereof.
**36.** The method of paragraph 22, wherein said inhibiting **IkBa** phosphorylation comprises administering a peptide fragment having an amino acid sequence comprising Ac-Ile-Glu-Gln-Glu-Lys-Gln-Ala-Gly-Glu-Ser-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Ac-Ile-Glu-Gln-Glu-Lys-Gln-Ala-Gly-Glu-Ser-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser or a combination thereof.
**37.** The method of paragraph 22, wherein said restoring impaired T-lymphocyte blastogenic response comprises administering a peptide fragment having an amino acid sequence comprising H-Leu-Lys-Lys-Thr-Glu-Thr-Gln-Glu-Lys-Asn-Pro-Leu-Pro-Ser-Lys-Glu-Thr-lle-Glu-Gln-Glu-Lys-Gln-Ala-Gly-Glu-Ser-OH, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser-OH, H-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser, Ac-Leu-Lys-Lys-Thr-Glu-Thr-Gln-Glu-Lys-Asn-Pro-Leu-Pro-Ser-Lys-Glu-Thr-Ile-Glu-Gln-Glu-Lys-Gln-Ala-Gly-Glu-Ser or a combination thereof.
**38.** The fragment of paragraph 1, wherein said fragment corresponds to a methionine-containing fragment, but has an amino acid substituent substituted for at least one methionine of the fragment, said substituent comprising leucine, valine, isoleucine, alanine, phenylalanine or proline, substituted for said methionine.

## Claims

1. A peptide fragment for use in a method of protecting tissue of a subject from cytotoxicity and/or inhibiting collagens in tissue of a subject, wherein said fragment has an amino acid sequence that consists of Ac-Ser-Asp-Lys-Pro-Asp-Met-Ala-Glu-Ile-Glu-Lys-Phe-Asp-Lys-Ser-OH (SEQ ID NO: 14).

2. A composition for the use claimed in claim 1, the composition comprising the peptide fragment as defined in claim 1 and a pharmaceutically acceptable carrier.


SEQUENCE LISTING

<110>  Regenerx Biopharmaceuticals, Inc.

<120>  Improved Beta Thymosin Fragments

<130>  K 84 357/7

<150>  US 61/083,798
<151>  2008-07-25

<150>  US 61/037,207
<151>  2008-03-17

<160>  22

<170>  PatentIn version 3.5

<210>  1
<211>  6
<212>  PRT
<213>  Homo sapiens

<220>
<221>  MISC_FEATURE
<222>  (1)..(1)
<223>  Hydrogen-  has the effect that the polypeptide suppresses
       inflammation

<400>  1

Leu Lys Lys Thr Glu Thr
1               5

<210>  2
<211>  4
<212>  PRT
<213>  Homo sapiens

<220>
<221>  MISC_FEATURE
<222>  (1)..(1)
<223>  hydrogen- has effect that the polypeptide inhibits collagens and
       stimulates collagen IV.

<400>  2

Ser Asp Lys Pro
1

<210>  3
<211>  15
<212>  PRT
<213>  Homo sapiens

<220>
<221>  MISC_FEATURE
<222>  (1)..(1)

<223>  hydrogen- has the effect that the polypeptide suppresses
       inflammation and inhibits apoptosis.

<400>  3

Ser Asp Lys Pro Asp Met Ala Glu Ile Glu Lys Phe Asp Lys Ser
1               5                   10                  15


<210>  4
<211>  7
<212>  PRT
<213>  Homo sapiens


<220>
<221>  MISC_FEATURE
<222>  (1)..(1)
<223>  hydrogen- has the effect that the polypeptide stimulates collagen
       IV and inhibits NFkB translocation.

<400>  4

Leu Lys Lys Thr Glu Thr Gln
1               5


<210>  5
<211>  10
<212>  PRT
<213>  Homo sapiens


<220>
<221>  MISC_FEATURE
<222>  (1)..(1)
<223>  hydrogen- has the effect that the polypeptide stimulates
       migration.

<400>  5

Glu Lys Asn Pro Leu Pro Ser Lys Glu Thr
1               5                   10


<210>  6
<211>  10
<212>  PRT
<213>  Homo sapiens


<220>
<221>  MISC_FEATURE
<222>  (1)..(1)
<223>  hydrogen- has the effect that the polypeptide inhibits collagens.

<400>  6

Ile Glu Gln Glu Lys Gln Ala Gly Glu Ser
1               5                   10


<210>  7

<211>  10
<212>  PRT
<213>  Homo sapiens

<220>
<221>  MISC_FEATURE
<222>  (1)..(1)
<223>  hydrogen- has the effect that the polypeptide inhibits collagens.

<400>  7

Met Ala Glu Ile Glu Lys Phe Asp Lys Ser
1               5                   10

<210>  8
<211>  22
<212>  PRT
<213>  Homo sapiens

<220>
<221>  MISC_FEATURE
<222>  (1)..(1)
<223>  hydrogen- has the effect that the polypeptide stimulates collagen
       IV and inhibits NFkB translocation.

<400>  8

Ser Asp Lys Pro Asp Met Ala Glu Ile Glu Lys Phe Asp Lys Ser Lys
1               5                   10                  15

Leu Lys Lys Thr Glu Thr
            20

<210>  9
<211>  23
<212>  PRT
<213>  Homo sapiens

<220>
<221>  MISC_FEATURE
<222>  (1)..(1)
<223>  hydrogen- has the effect that the polypeptide stimulates collagen
       IV and inhibits NFkB translocation.

<400>  9

Ser Asp Lys Pro Asp Met Ala Glu Ile Glu Lys Phe Asp Lys Ser Lys
1               5                   10                  15

Leu Lys Lys Thr Glu Thr Gln
            20

<210>  10
<211>  25
<212>  PRT

<213>  Homo sapiens

<220>
<221>  MISC_FEATURE
<222>  (1)..(1)
<223>  hydrogen- has the effect that the polypeptide stimulates collagen
       IV and inhibits NFkB translocation

<400>  10

Ser Asp Lys Pro Asp Met Ala Glu Ile Glu Lys Phe Asp Lys Ser Lys
1               5                   10                  15

Leu Lys Lys Thr Glu Thr Gln Glu Lys
            20                  25

<210>  11
<211>  27
<212>  PRT
<213>  Homo sapiens

<220>
<221>  MISC_FEATURE
<222>  (1)..(1)
<223>  hydrogen- has the effect that the polypeptide inhibits collagens
       and stimulates elastin.

<400>  11

Leu Lys Lys Thr Glu Thr Gln Glu Lys Asn Pro Leu Pro Ser Lys Glu
1               5                   10                  15

Thr Ile Glu Gln Glu Lys Gln Ala Gly Glu Ser
            20                  25

<210>  12
<211>  6
<212>  PRT
<213>  Homo sapiens

<220>
<221>  MISC_FEATURE
<222>  (1)..(1)
<223>  acetyl- has the effect that the polypeptide inhibits collagen.

<400>  12

Leu Lys Lys Thr Glu Thr
1               5

<210>  13
<211>  4
<212>  PRT
<213>  Homo sapiens

<220>
<221>  MISC_FEATURE
<222>  (1)..(1)
<223>  acetyl- has the effect that the polypeptide suppresses
       inflammation and stimulates migration.

<400>  13

Ser Asp Lys Pro
1


<210>  14
<211>  15
<212>  PRT
<213>  Homo sapiens


<220>
<221>  MISC_FEATURE
<222>  (1)..(1)
<223>  acetyl- has the effect that the polypeptide protects from
       cytotoxicity and inhibits collagens.

<400>  14

Ser Asp Lys Pro Asp Met Ala Glu Ile Glu Lys Phe Asp Lys Ser
1               5                   10                  15


<210>  15
<211>  7
<212>  PRT
<213>  Homo sapiens


<220>
<221>  MISC_FEATURE
<222>  (1)..(1)
<223>  acetyl- has the effect that the polypeptide stimulates migration.

<400>  15

Leu Lys Lys Thr Glu Thr Gln
1               5


<210>  16
<211>  10
<212>  PRT
<213>  Homo sapiens


<220>
<221>  MISC_FEATURE
<222>  (1)..(1)
<223>  acetyl- has the effect that the polypeptide stimulates collagens.

<400>  16

Glu Lys Asn Pro Leu Pro Ser Lys Glu Thr
1               5                   10

<210>  17
<211>  10
<212>  PRT
<213>  Homo sapiens

<220>
<221>  MISC_FEATURE
<222>  (1)..(1)
<223>  acetyl- has the effect that the polypeptide inhibits IkBa
       phosphorylation.

<400>  17

Ile Glu Gln Glu Lys Gln Ala Gly Glu Ser
1               5                   10

<210>  18
<211>  10
<212>  PRT
<213>  Homo sapiens

<220>
<221>  MISC_FEATURE
<222>  (1)..(1)
<223>  acetyl- has the effect that the polypeptide stimulates elastin.

<400>  18

Met Ala Glu Ile Glu Lys Phe Asp Lys Ser
1               5                   10

<210>  19
<211>  22
<212>  PRT
<213>  Homo sapiens

<220>
<221>  MISC_FEATURE
<222>  (1)..(1)
<223>  acetyl- has the effect that the polypeptide stimulates collagens.

<400>  19

Ser Asp Lys Pro Asp Met Ala Glu Ile Glu Lys Phe Asp Lys Ser Lys
1               5                   10                  15

Leu Lys Lys Thr Glu Thr
            20

<210>  20
<211>  23
<212>  PRT
<213>  Homo sapiens

<220>
<221>  MISC_FEATURE
<222>  (1)..(1)
<223>  acetyl- has the effect that the polypeptide stimulates collagens.

<400>  20

Ser Asp Lys Pro Asp Met Ala Glu Ile Glu Lys Phe Asp Lys Ser Lys
1               5                   10                  15

Leu Lys Lys Thr Glu Thr Gln
            20

<210>  21
<211>  25
<212>  PRT
<213>  Homo sapiens

<220>
<221>  MISC_FEATURE
<222>  (1)..(1)
<223>  acetyl -has the effect that the polypeptide stimulates collagens.

<400>  21

Ser Asp Lys Pro Asp Met Ala Glu Ile Glu Lys Phe Asp Lys Ser Lys
1               5                   10                  15

Leu Lys Lys Thr Glu Thr Gln Glu Lys
            20                  25

<210>  22
<211>  27
<212>  PRT
<213>  Homo sapiens

<220>
<221>  MISC_FEATURE
<222>  (1)..(1)
<223>  acetyl- has the effect that the polypeptide inhibits IkBa
       phosphorylation.

<400>  22

Leu Lys Lys Thr Glu Thr Gln Glu Lys Asn Pro Leu Pro Ser Lys Glu
1               5                   10                  15

Thr Ile Glu Gln Glu Lys Gln Ala Gly Glu Ser
            20                  25